# EUROPÄISCHE PATENTSCHRIFT

(11) **EP 3 308 191 B1**
(45) Veröffentlichungstag und Bekanntmachung des Hinweises auf die Patenterteilung: **24.07.2019**
(21) Anmeldenummer: 16739010.3
(22) Anmeldetag: 09.06.2016
(51) Int. Cl.: G01S 5/02

(54) **VERFAHREN FÜR DIE ORTUNG VON TIEREN MIT HILFE VON FUNKWELLEN**
METHOD FOR LOCATING ANIMALS USING RADIO WAVES
PROCÉDÉ POUR LA LOCALISATION D'ANIMAUX À L'AIDE D'ONDES RADIO

(30) Priorität: 12.06.2015 AT 3742015
(43) Veröffentlichungstag der Anmeldung: 18.04.2018
(73) Patentinhaber: Smartbow GmbH, 4675 Weibern (AT)
(72) Erfinder: AUER, Wolfgang, 4675 Weibern (AT); RUDIC, Branislav, 4020 Linz (AT); PICHLER, Markus, 4532 Kematen (AT)
(86) Internationale Anmeldenummer: PCT/AT2016/000064
(87) Internationale Veröffentlichungsnummer: WO 2016/197160

(56) Entgegenhaltungen:
- EP-A1- 1 109 031
- US-A- 6 113 539
- US-A1- 2013 035 110

## Beschreibung

Die Erfindung betrifft ein Verfahren für die Ortung von Tieren mit Hilfe von Funkwellen.

Übliche Anlagen für die Ortung von Objekten, wie beispielsweise Tieren, mit Hilfe von Funkwellen, weisen mehrere Funksender und/oder Funkempfänger auf die sich an bekannten Positionen befinden, sowie jeweils einen Funksender und/oder Funkempfänger an jedem zu ortenden Objekt.

Des Weiteren werden die Sender bzw. Empfänger, welche für die Ortung mittels Funkwellen verwendet werden einfach als "Knoten" bezeichnet. Entsprechend einem häufig angewandten Verfahren wird für das Bestimmen der Position des an dem zu ortenden Tier angebrachten Knotens im ersten Schritt mit Hilfe von Funksignalen gemessen, um welche Länge sich die Abstände der einzelnen Knoten bekannter Position zu dem zu ortenden Knoten voneinander unterscheiden. Beispielsweise wird dazu von dem zu ortenden Knoten aus ein Signal gleichzeitig an alle anderen Knoten abgesandt. Die empfangenden Knoten messen den Zeitpunkt zu welchem dieses Signal bei ihnen ankommt. Die Differenzen zwischen den einzelnen gemessenen Zeitpunkten, jeweils multipliziert mit der Lichtgeschwindigkeit (Signalausbreitungsgeschwindigkeit im betreffenden Medium) ergeben die Abstandsunterschiede der einzelnen Knoten zum sendenden Knoten. Für die weitere Berechnung wird im ersten Schritt davon ausgegangen, dass sich der zu ortende Knoten auf einem Hyperboloid befindet, dessen Achse durch zwei Knoten bekannter Position als Brennpunkte verläuft, wobei der gemessene Abstandsunterschied dieser Knoten zu dem zu ortenden Knoten gleich jener Länge ist, um welche sich - definitionsgemäß - die Distanzen zwischen den beiden Brennpunkten zu jedem auf dem Hyperboloid befindlichen Punkt unterscheiden. Durch Schneiden von mindestens drei derartigen Hyperboloiden wird die mögliche Position des zu ortenden Gerätes auf zwei Punkte eingeschränkt. Die weitere Einschränkung auf einen Punkt kann mit Hilfe eines vierten Hyperboloids erfolgen (sodass also mindestens vier Knoten bekannter Position erforderlich sind) oder indem an Hand von bekannten geometrischen Gegebenheiten ein Punkt ohnedies ausgeschlossen werden kann, beispielsweise weil er sich außerhalb des Stalls innerhalb dessen sich Tiere bewegen können, befindet. (Mit "Hyperboloid" ist in diesem Text eine rotationssymmetrische schalenförmige Fläche gemeint, die als durch Rotation einer Hyperbel um ihre Hauptachse entstanden gedacht werden kann.)

Wenn in einer Abwandlung zu dem beschriebenen Verfahren im ersten Schritt mittels Funkwellen direkt auf die Abstände zwischen dem Knoten an dem zu ortenden Tier zu den einzelnen Knoten bekannter Position geschlossen wird, so sind im zweiten Schritt anstatt der Hyperboloid-Schalen Kugelschalen anzunehmen.

Mit Funklokalisierung von Tieren entsprechend dem erklärten Prinzip befassen sich beispielsweise die Schriften AT 506628 A1, US 6122960 A, DE 100 45 469 C2, WO9941723 A1 WO2011153571 A2 und WO2012079107 A2.

Auf Grund von in der Praxis oftmals unvermeidbar vorkommenden erheblichen Messfehlern und Messungenauigkeiten - z.B. wegen Reflexionen von Funkwellen - sind weitere logische Annahmen einzubringen und dementsprechende Auswertungen vorzunehmen, um ein einigermaßen verlässliches Ortungsergebnis erhalten zu können. Neben dem schon erwähnten Ausschließen von Ergebnissen die auf Grund geometrischer Gegebenheiten unmöglich sind, werden vor allem stochastische Methoden herangezogen um auch an Hand der Ergebnisse vorhergegangener Messungen die Vieldeutigkeit des/der jeweils aktuellen Ergebnisse einzuschränken und jenes Messergebnis zu finden, welches mit der geringsten Fehlerwahrscheinlichkeit die Wirklichkeit widerspiegelt. Ein bewährtes stochastisches Modell ist in diesem Zusammenhang das Hidden-Markov-Model und dabei insbesondere der Viterbi-Algorithmus, mit Hilfe dessen relativ effizient aus einer Vielzahl von möglichen Zustandsfolgen die aktuell jeweils wahrscheinlichste Zustandsfolge gefunden werden kann.

Eine üblicherweise für die Berechnung der Aufenthaltswahrscheinlichkeit angewandte Randbedingung ist, dass zumindest ab einer gewissen Grenzdistanz zwischen einem aktuell in Prüfung befindlichen Ort und dem zuletzt angenommen Aufenthaltsort, mit steigender Distanz die Wahrscheinlichkeit sinkt, dass der aktuell in Prüfung befindliche Ort der aktuelle Aufenthaltsort ist. Etwas vereinfacht ausgedrückt heißt das, dass ein aktueller Aufenthaltsort eines Tieres mit hoher Wahrscheinlichkeit nur innerhalb eines Kreises mit bestimmtem, begrenztem Radius vom letzten vorher angenommenen Aufenthaltsort liegen kann.

Beispielsweise die EP 1 494 397 A2 beschreibt eine Methode der Funklokalisierung insbesondere für die Anwendung in Gebäuden. In Gebäuden ist die Funklokalisierung wegen vielfach vorkommenden Signalreflexionen besonders schwierig.

Beispielsweise in den Schriften EP 549081 A1, GB 2234070 A, GB 2278198 A, US 3999611 A, US 6122960 A, US 7616124 B2, WO 2002091001 A1, WO 2003055388 A2, WO 2006077589 A2, WO 2010108496 A1 und WO 2010109313 A1 wird vorgeschlagen und erläutert Beschleunigungssensoren (unter anderem) an lebendigen Tieren anzubringen und aus den Messergebnisse der Beschleunigungssensoren auf das Verhalten der Tiere zurückzuschließen welches zu den jeweiligen Beschleunigungen führt. Üblicherweise werden dazu die Messergebnisse über eine Funkverbindung an eine Datenverarbeitungsanlage übertragen und durch diese auf Übereinstimmungen mit als Muster gespeicherten zeitlichen Verläufen von Beschleunigungsdaten überprüft.

Die als Muster gespeicherten zeitlichen Verläufe sind dabei charakteristisch für bestimmte Aktivitäten des Tieres wie beispielsweise Gehen, Fressen, Wiederkäuen, Schlafen, ggf. hinkendes Gehen, Aufreiten auf andere Tiere. Für das Finden der charakteristischen Muster wurden in früheren Arbeiten Beschleunigungsdaten und parallel dazu die an Hand von unmittelbarer Beobachtung festgestellten Aktivitäten von Tieren aufgezeichnet und aus den aufgezeichneten Daten Korrelationen zwischen Beschleunigungsmustern und Aktivitäten herausgefiltert.

Die WO9941723 A1 befasst sich mit einem von einem Menschen oder Tier mitgeführten Gerät, welches Funkwellen senden und empfangen kann und dessen Position durch ein Satellitennavigationssystem feststellbar ist. Es wird auch erwähnt, dass das Gerät neben verschiedenen anderen Sensoren die beispielsweise einen biologischen Zustand messen können, auch einen Beschleunigungssensor aufweisen kann.

Die WO2011153571 A2 und die WO2012079107 A2 befassen sich mit funkfähigen Ohrmarken für Tiere, wobei eine Ohrmarke sowohl Funkortung ermöglicht als auch einen Beschleunigungssensor enthalten kann, mit Hilfe dessen durch Musterauswertung Aktivitäten des Tieres automatisch erkennbar sind.

Die US 6113539 A beschreibt ein Verfahren für die Ortung eines Tieres, welches die Ortung von einem Knoten eines Funksystems anhand Abstandmessungen zwischen dem zu ortenden Knoten und dem bekannten Knoten des Systems offenbart.

Die US 6122960 A befasst sich vorwiegend mit der Messung und Aufzeichnung von Bewegungen und zurückgelegten Entfernungen von Menschen oder Tieren durch Messung von Beschleunigungen und Auswertung der Messungen (zweifache Integration der gemessenen Beschleunigungsvektoren über die Zeit). Es wird vorgeschlagen ergänzend eine "Absolutposition" durch Funknavigation festzustellen.

Von diesem Stand der Technik ausgehend liegt die Aufgabe an die Erfindung darin, ein auch in Laufställen und Koppeln für Tiere anwendbares automatisch ablaufendes Verfahren für die Ortung von Tieren mit Hilfe von Funkwellen bereitzustellen, welches gegenüber bekannten derartigen Verfahren bezogen auf den dafür erforderlichen Investitionsaufwand genauere und besser verlässliche Ergebnisse liefert.

Zum Lösen der Aufgabe wird von der bekannten Methode der Funkortung ausgegangen, wonach stochastische Berechnungen angewandt werden, um an Hand der Ergebnisse vorhergegangener Messungen und Berechnungen bezüglich der möglichen Position des zu ortenden Knotens, aus der Mehrzahl der jeweils aktuellen Positions-Berechnungsergebnisse jenes Ergebnis herauszufiltern, welches mit der höchsten Wahrscheinlichkeit (also mit der geringsten Fehlerwahrscheinlichkeit) tatsächlich die aktuelle Position beschreibt, wobei als eine Randbedingung mit einfließt, dass zumindest ab einer gewissen Grenzdistanz zwischen der durch ein aktuelles Berechnungsergebnis definierten Position und der zuletzt angenommenen Position des zu ortenden Knotens, mit steigender Distanz zwischen den beiden Positionen, die Wahrscheinlichkeit sinkt, dass die Position gemäß dem aktuellen Berechnungsergebnis die tatsächliche Position des zu ortenden Knotens ist.

Erfindungsgemäß wird vorgeschlagen, das Verfahren zu verbessern, indem auch Beschleunigungsdaten am zu ortenden Tier gemessen werden und die gemessenen Beschleunigungsdaten derart in die Wahrscheinlichkeit miteinfließen, dass dann wenn höhere Beschleunigungswerte gemessen werden, die Wahrscheinlichkeit für größere Distanz zwischen zwei zeitlich nacheinander eingenommenen Aufenthaltsorten zu Ungunsten der Wahrscheinlichkeit für kleinere Distanz zwischen zwei zeitlich nacheinander eingenommenen Aufenthaltsorten, als vergrößert angenommen wird.

Die Erfindung wird unter Zuhilfenahme von Zeichnung zu einer vorteilhaften beispielhaften erfindungsgemäßen Verfahrensvariante verdeutlich:
- Fig. 1:: veranschaulicht stark stilisiert, die Aufgabenstellung aus vielen errechneten Aufenthaltsorten jene Folge von Aufenthaltsorten herauszufinden, die mit der höchsten Wahrscheinlichkeit der Wirklichkeit entspricht.
- Fig. 2:: zeigt in kartesischer Koordinatendarstellung zwei angenommene Wahrscheinlichkeitsverläufe a, b für die Größe von Distanzen zwischen zeitlich aufeinanderfolgend durch Messung und Berechnung gefundenen möglichen Positionen (Aufenthaltsorten) des zu ortenden Knotens.

Ein Funkortungssystem für das Feststellen des Aufenthaltsortes und der Bewegungen eines Tieres weist - wie weiter oben näher erklärt - mehrere Knoten auf, wobei das Tier, welches sich frei bewegen kann, den zu ortenden Knoten trägt und eine Mehrzahl von weiteren Knoten unbeweglich montiert sind und ihre relative Lage zueinander bekannt ist. In geregelten zeitlichen Abständen wird ein Mess- und Berechnungsablauf für die Funkortung durchgeführt. Wie weiter oben beschrieben, ist die Messung entweder eine Abstandsmessungen zwischen dem zu ortenden Knoten und den Knoten bekannter Position oder die Messung der Differenzen der Abstände zwischen dem zu ortenden Knoten und den einzelnen Knoten bekannter Position. Aus den Messergebnissen wird durch geometrische Berechnung auf die mögliche Position des zu ortenden Knotens (relativ zu den Knoten bekannter Position) zurückgeschlossen. Auf Grund dessen, dass sehr oft mehr als vier Knoten bekannter Position vorliegen und dass Funksignale zwischen zwei Knoten im Normalfall nicht nur in direkter gerader Linie übertragen werden, sondern zufolge Reflexion zusätzlich über weitere, längere Wege, fällt eine Vielzahl von Messereignissen an die man mathematisch als ein überbestimmtes, in sich widersprüchliches System bezeichnen kann. Konkret heißt das, dass man dann, wenn man immer die jeweils letzten vier Empfangsereignisse von Funksignalen zwischen dem zu ortenden Knoten und jeweils einem anderen Knoten bekannter Position zur Berechnung der Position des zu ortenden Knotens verwendet, eine Vielzahl von Berechnungsergebnissen erhält, die jeweils eine Position im Raum beschreiben, wobei aber nur ein kleiner Teil dieser Berechnungsergebnisse tatsächlich die Positionen des zu ortenden Knotens beschreiben. Die überwiegende Anzahl der Berechnungsergebnisse hingegen ist falsch.

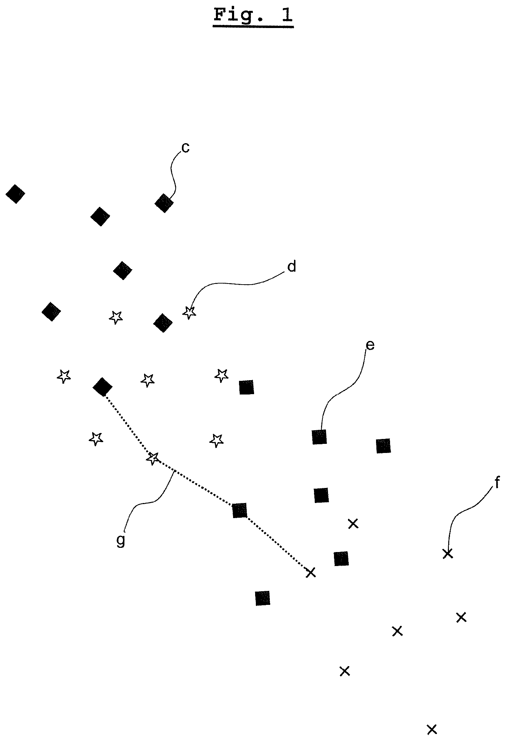

In Fig. 1 ist dieser Zusammenhang symbolisiert. Auf eine Mehrzahl von Berechnungsergebnissen die jeweils eine Position c (jeweils durch ein schräg stehendes Quadrat symbolisiert) bedeuten, folgt eine Mehrzahl an Berechnungsergebnissen die jeweils eine Position d (jeweils durch einen Stern symbolisiert) bedeuten, usw. über Berechnungsergebnisse zu Positionen e und Positionen f.

Von vornherein kann man als feststehend annehmen, dass auf dem tatsächlichen Weg des zu ortenden Knotens auf eine einzige der Positionen c eine einzige der Positionen d folgt, dann eine einzige der Positionen e und schließlich eine einzige der Positionen f. Stellvertretend für die Unzahl von damit prinzipiell möglichen Wegen ist in Fig. 1 beispielhaft ein einzelner Weg g durch eine punktierte Linie symbolisiert.

Zur Berechnung, welcher Weg tatsächlich am wahrscheinlichsten die Wirklichkeit wiederspiegelt, zieht man in Betracht, dass die möglichen Distanzen zwischen zwei zeitlich unmittelbar hintereinander folgenden Positionen (c und d, d und e, e und f) nicht allesamt gleich wahrscheinlich sind, sondern dass manche Klassen von Distanzen mehr wahrscheinlich sind und andere weniger. Extrem große Distanzen beispielsweise sind unmöglich, weil sie bedeuten würden, dass sich das Tier, welches den zu ortenden Knoten trägt, schneller bewegen müsste als es die Grenzen von Biologie und Technik zulassen.

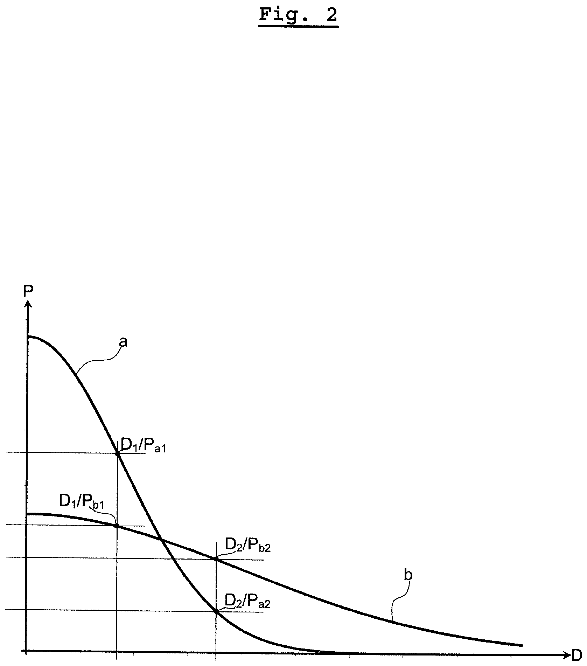

Fig. 2 zeigt ein Diagramm welches Aussagen enthält bezüglich der Wahrscheinlichkeit von Beträgen von Distanzen zwischen zwei zeitlich direkt hintereinander liegenden Positionen.

Die beiden Kurven a, b beschreiben jeweils, wie sehr eine Distanz D1, D2 zwischen zwei zeitlich aufeinanderfolgenden Positionen wahrscheinlich ist.

Wenn z. B. Kurve a gilt und für einen Zeitpunkt genau zwei mögliche Distanzen D1 und D2 errechnet werden, so zeigt das Größenverhältnis der den Distanzen D1 bzw. D2 zugeordneten Ordinatenmaße Pa1 und Pa2 zueinander das Verhältnis der Wahrscheinlichkeit das D1 zutrifft zur Wahrscheinlichkeit das D2 zutrifft. Für die weiterführende Berechnung muss die absolute Größe der Wahrscheinlichkeiten noch so normiert werden, dass die Summe der Wahrscheinlichkeiten über alle möglichen Positionen immer den gleichen Wert ergibt (am besten 1).

Beide Kurven a und b haben das Maximum bei D=0, womit gemäß beiden Kurven Distanzen zwischen zeitlich aufeinander folgenden Positionen umso wahrscheinlicher sind, umso kleiner die Distanzen sind.

Kurve a fällt aber deutlich steiler ab als Kurve b. Das bedeutet, dass dann wenn Kurve a zur Annahme der Wahrscheinlichkeit dient, die Wahrscheinlichkeit dass die größere Distanz D2 die Zutreffende ist, gegenüber der Wahrscheinlichkeit dass die kleinere Distanz D1 die Zutreffendere ist, sehr viel geringer ist, als wenn Kurve b zur Annahme der Wahrscheinlichkeit dient.

Erfindungsgemäß ist vorgesehen, dass die Auswahl, welche Kurve a oder b verwendet wird, davon abhängig ist, welche Beschleunigungen an dem vom Tier mitgeführten Beschleunigungssensor gemessen werden. Wenn während der Zeitperiode für welche die Position zu berechnen ist, kleine Beschleunigungswerte gemessen wurden, wird die steilere Kurve a herangezogen, wenn größere Beschleunigungswerte gemessen wurden, wird Kurve b herangezogen.

Die Kurven a, b können beispielsweise als eine Normalverteilung nach Gauß angenommen sein, wobei die in die Berechnungsformel der Kurven bekanntermaßen miteinfließende angenommene Standardabweichung als von der gemessenen Beschleunigung abhängig angenommen werden kann. Die Abhängigkeitsfunktion sollte bevorzugt so sein, dass mit steigender Beschleunigung die Standardabweichung monoton mit steigt, beispielsweise direkt proportional zur gemessenen Beschleunigung mit steigt.

Optimale Kurvenverläufe und Abhängigkeiten wie die Form der Kurven von den gemessenen Beschleunigungen abhängig sind, müssen empirisch ermittelt werden. Dazu werden Bewegungsabläufe von Tieren - oder Robotern etc. - einerseits durch direkte Beobachtung, Wegmessung und Aufzeichnung protokolliert und andererseits wie beschrieben durch Funkortung, Beschleunigungsmessungen und zusammenführende Berechnung ermittelt. Die zusammenführende Berechnung wird mit unterschiedlichen zu Grunde liegenden Berechnungsparametern (z.B. unterschiedliche Abhängigkeiten der Standardabweichungen von Kurven gemäß Fig. 1 von der gemessenen Beschleunigung) so lange variiert, bis die beiden Ermittlungsmethoden bestmöglich gleich gute Ergebnisse liefern.

Wenn alle Berechnungsparameter richtig angenommen wurden und in die Berechnung miteinfließen, kann der beste der möglichen Wege g gemäß Fig. 1 gefunden werden, indem von allen möglichen Wegen entlang Positionsfolgen c-d-e-f die jeweiligen Produkte der jeweils drei Wahrscheinlichkeiten gebildet werden die den jeweiligen Distanzen c-d, d-e und e-f gemäß Fig. 1 zugeordnet sind und dann jener Weg ausgewählt wird bei welchem dieses Produkt am höchsten ist. (Um den Rechenaufwand in gut erträglichen Grenzen zu halten sollten die eingangs erwähnten bekannten Methoden Hidden-Markov-Model insbesondere Viterbi-Algorithmus angewendet werden.)

Unter dem in die Berechnung der Wahrscheinlichkeiten eingehenden Beschleunigungswert ist genaugenommen ein mathematisch aufbereiteter Zahlenwert zu verstehen, der aus der Mehrzahl der in der jeweils aktuellen Zeitperiode gemessenen Beschleunigungswerte gebildet wird und diese Mehrzahl von Werten gut repräsentiert. Es kann ein statistischer Mittelwert wie beispielsweise der Effektivwert (quadratischer Mittelwert) sein, oder die geometrische Summe der in einzelnen Richtungen gemessenen Effektivwerte oder der arithmetische Mittelwert oder der Mittelwert der einzelnen Absolutwerte, etc. sein, es kann aber auch eine gewichtete Mischung verschiedener derartiger Mittelwerte sein oder der Betrag einer vektoriellen Summe der einzelnen festgestellten Beschleunigungsvektoren. In der Praxis ermittelt man die diesbezügliche Festlegungsmethode wiederum am besten empirisch. Die Auswahl ist nicht nur von der theoretischen mathematischen Präzision sondern beispielsweise auch von der Messfrequenz, der Messgenauigkeit, der verfügbaren Rechenkapazität etc. abhängig.

Als Ergebnis der Überwachung der Bewegungen eines Tieres ist vor allem interessant, wieviel Weg das Tier in einer Zeitperiode (z.B. in einem Tag) zurückgelegt hat, weil das sehr viel über den Grad der Aktivität des Tieres und damit über den Zustand des Tieres aussagt. Die Kenntnis der exakten Positionen die das Tier zu den einzelnen Zeitpunkten innehatte, ist hingegen weniger aussagekräftig und damit auch weniger interessant.

Vor allem wenn es um die Überwachung des gesamten Weges, den ein Tier in einer "längeren Zeitperiode" (z.B. eine Stunde oder ein Tag) zurücklegt geht, wirken sich Fehler der Funkortung relativ gesehen sehr stark aus, wenn das Tier wenig Bewegung macht und damit auch geringe Beschleunigungen gemessen werden.

Daher ist es gemäß einer bevorzugten Weiterentwicklung des erfindungsgemäßen Verfahrens zu empfehlen, in die Berechnung der wahrscheinlichsten Trajektorie die ein an einem Tier befestigter Knoten eines Funkortungssystems zurücklegt, mit einfließen zu lassen, dass Ergebnisse des Funkortungssystems - also die durch Funksignalübertragung gewonnenen Informationen über den Abstand des zu ortenden Knotens von Knoten bekannter Position bzw. über den Abstand zwischen zeitlich aufeinanderfolgenden Positionen des zu ortenden Knotens - die gewonnen wurden, während hohe Beschleunigungswerte gemessen wurden, mit höherer Wahrscheinlichkeit zutreffend sind, als Ergebnisse des Funkortungssystems die gewonnen wurden, während niedrige Beschleunigungswerte gemessen wurden.

Es gibt eine Vielzahl von Möglichkeiten, das in die konkrete Berechnung der als am wahrscheinlichsten anzusehenden Trajektorie einzubringen. Als extrem einfache beispielhafte Methode kann man Messergebnisse der Funkortung, die für Zeitbereiche gelten an denen der Betrag der gemessenen Beschleunigung unterhalb einer bestimmten Mindestgrenze lag, einfach ignorieren. Die letztendlich angenommene Trajektorie verläuft dann auf direktmöglichstem Weg nur zwischen jenen möglichen Ergebnispositionen der Funkortung die für Zeitpunkte gelten bei denen Beschleunigungen gemessen wurden die über dem besagten Mindestwert liegen.

## Patentansprüche

1. Verfahren für die Ortung eines Tieres mit Hilfe von Funkwellen wobei das Tier mit einem zu ortenden Knoten des Funkortungssystem ausgestattet ist und wobei mehrere weitere Knoten des Funkortungssystems an bekannten Positionen angeordnet sind, wobei Funksignale zwischen dem zu ortenden Knoten und den Knoten mit bekannter Position übertragen werden und aus bei der Signalübertragungen gemessenen Parametern einzelne Abstände und/oder einzelne Abstandsunterschiede zwischen den einzelnen Knoten bekannter Position einerseits und dem zu ortenden Knoten andererseits errechnet werden und durch eine Datenverarbeitungsanlage aus mehreren derartiger Berechnungsergebnisse mögliche Positionen des zu ortenden Knotens errechnet werden,
**dadurch gekennzeichnet, dass**
stochastische Berechnungen angewandt werden, um an Hand der Ergebnisse vorhergegangener Messungen und Berechnungen bezüglich der möglichen Position des zu ortenden Knotens, aus der Mehrzahl der jeweils aktuellen Positions-Berechnungsergebnisse jenes Ergebnis herauszufiltern, welches mit maximaler Wahrscheinlichkeit tatsächlich die aktuelle Position beschreibt, wobei als eine Randbedingung mit einfließt, dass zumindest ab einer gewissen Grenzdistanz zwischen der durch ein aktuelles Berechnungsergebnis definierten Position und der zuletzt angenommen Position des zu ortenden Knotens, mit steigender Distanz zwischen den beiden Positionen, die Wahrscheinlichkeit sinkt, dass die Position gemäß dem aktuellen Berechnungsergebnis die tatsächliche Position des zu ortenden Knotens ist, wobei auch Beschleunigungsdaten an dem mit dem zu ortenden Knoten ausgestatteten Tier gemessen werden und die gemessenen Beschleunigungsdaten an die Datenverarbeitungsanlage übertragen werden, wobei die Annahme der besagten Wahrscheinlichkeit von den gemessenen Beschleunigungsdaten abhängt, wobei bei größeren gemessenen Beschleunigungswerten die Wahrscheinlichkeit für größere Distanz zwischen zwei zeitlich nacheinander eingenommenen Positionen zu Ungunsten der Wahrscheinlichkeit für kleinere Distanz zwischen zwei zeitlich nacheinander eingenommenen Positionen, als vergrößert angenommen wird.

2. Verfahren nach Anspruch 1, **dadurch gekennzeichnet, dass** die Wahrscheinlichkeit für die Größe einer Distanz zwischen zwei zeitlich aufeinander folgenden Positionen des zu ortenden Knotens in Abhängigkeit vom Betrag der Distanz als zumindest annähernd einer Normalverteilung nach Gauß folgend angenommen wird, wobei die Standardabweichung als mit der gemessenen Beschleunigung monoton steigend angenommen wird.

3. Verfahren nach Anspruch 1 oder 2, **dadurch gekennzeichnet, dass** als der in die Annahme der Wahrscheinlichkeit einfließende Beschleunigungswert ein statistischer Mittelwert von zeitlich aufeinanderfolgenden Messwerten von Beschleunigungen verwendet wird.

4. Verfahren nach einem der Ansprüche 1 bis 3, **dadurch gekennzeichnet, dass** bei der Berechnung der wahrscheinlichsten Abfolge einer Reihe von Positionen die der zu ortende Knoten eingenommen hat, sich aus der Funksignalübertragung gemessene Abstandsinformationen bezüglich des zu ortenden Knotens mit höherer Wahrscheinlichkeit als zutreffend in die Berechnung mit einfließen, wenn bei der Beschleunigungsmessung höhere Beschleunigungswerte festgestellt werden als wenn bei der Beschleunigungsmessung niedrigere Beschleunigungswerte festgestellt werden.

## Claims

1. A method for locating an animal by means of radio waves, wherein the animal is provided with a node of a radio wave location system to be located and wherein a plurality of further nodes of the radio wave location system are provided at known positions, wherein the radio wave signals are transmitted between the node to be located and the nodes having known positions and wherein individual distances and/or individual distance differences between the individual nodes having known positions on the one hand and the node to be located on the other hand are calculated from the parameters measured during the signal transmission and wherein, by a data processing device, possible positions of the node to be located are calculated from several such calculation results,
**characterised in that**
stochastic calculations are used in order to, based on the results of preceding measurements and calculations regarding the possible position of the node to be located, the one result out of the plurality of the current position calculation results is filtered out which with maximum likelihood actually describes the current position, wherein there is included the constraint that at least from a certain limiting distance between the position defined by a current calculation result and the latest assumed position of the node to be located, upon increasing distance between the two positions, the likelihood decreases that the position according to the current calculation result is the actual position of the node to be located, wherein there are also measured acceleration data on the animal provided with the node to be located and the measured acceleration data are transmitted to the data processing device,
wherein the assumption of said likelihood depends on the measured acceleration data, wherein, for greater measured acceleration values, the likelihood for greater distances between two temporally subsequently adopted positions is assumed to be increased at the detriment of the likelihood for a smaller distance between two temporally subsequently adopted positions.

2. Method according to claim 1, **characterised in that** the likelihood for the value of a distance between two temporally subsequent positions of the node to be located is assumed to be at least approximately distributed following a normal Gaussian distribution, depending on the modulus of the distance, wherein the standard deviation is assumed to increase monotonicaly with the measured acceleration.

3. Method according to claim 1 or 2, **characterised in that** the acceleration value taken into account in the assumption of the likelihood is a statistical average of temporally subsequent measurement values of accelerations.

4. Method according to one of claims 1 to 3, **characterised in that**, in the calculation of the most likely sequence of a series of positions which the node to be located has adopted, distance information regarding the node to be located measured from the radio wave transmission are taken into account with a higher likelihood of being accurate if, in the acceleration measurement, higher acceleration values are determined than if in the acceleration measurement lower acceleration values are determined.

## Revendications

1. Procédé pour la localisation d'un animal à l'aide d'ondes radio dans lequel l'animal est équipé d'un noeud à localiser du système de radiolocalisation et dans lequel plusieurs autres noeuds du système de radiolocalisation sont agencés à des positions connues, dans lequel des signaux radio sont transmis entre le noeud à localiser et les noeuds de position connue et différentes distances et/ou différentes différences de distance entre les différents noeuds de position connue d'un côté et le noeud à localiser d'un autre côté sont calculées à partir de paramètres mesurés lors des transmissions de signaux et de possibles positions du noeud à localiser sont calculées par une installation de traitement de données à partir de plusieurs résultats de calcul de ce type,
**caractérisé en ce que** des calculs stochastiques sont appliqués pour filtrer à l'aide des résultats de mesures et calculs précédents concernant la position possible du noeud à localiser, à partir de la pluralité des résultats de calcul de position respectivement courant, le résultat qui décrit effectivement la position courante avec une probabilité maximum, dans lequel il est pris en compte en tant que condition aux limites le fait qu'au moins à partir d'une certaine distance limite entre la position définie par un résultat de calcul courant et la dernière position adoptée du noeud à localiser la probabilité que la position conformément au résultat de calcul courant soit la position réelle du noeud à localiser diminue avec l'augmentation de la distance entre les deux positions,
dans lequel des données d'accélération sont aussi mesurées en l'animal équipé du noeud à localiser et les données d'accélération mesurées sont transmises à l'installation de traitement de données,
dans lequel l'hypothèse de ladite probabilité dépend des données d'accélération mesurées, dans lequel la probabilité d'une distance entre deux positions adoptées l'une après l'autre dans le temps plus grande est supposée augmenter au détriment de la probabilité d'une distance entre deux positions adoptées l'une après l'autre dans le temps plus petite, dans le cas de plus grandes valeurs d'accélération mesurées.

2. Procédé selon la revendication 1, **caractérisé en ce que** la probabilité pour la taille d'une distance entre deux positions se suivant dans le temps du noeud à localiser est supposée suivre au moins approximativement une distribution normale gaussienne en fonction de la valeur de la distance, dans lequel l'écart-type est supposé croissant de façon monotone avec l'accélération mesurée.

3. Procédé selon la revendication 1 ou 2, **caractérisé en ce qu'**une moyenne statistique de valeurs de mesure d'accélérations successives dans le temps est utilisée en tant que valeur d'accélération prise en compte dans l'hypothèse de la probabilité.

4. Procédé selon l'une des revendications 1 à 3, **caractérisé en ce que** lors du calcul de la suite la plus probable d'une série de positions que le noeud à localiser a adoptées, des informations de distance mesurées à partir de la transmission de signaux radio concernant le noeud à localiser sont prises en compte dans le calcul comme étant exactes avec une plus grande probabilité, lorsque des valeurs d'accélération plus élevées sont constatées lors de la mesure de l'accélération que lorsque des valeurs d'accélération plus faibles sont constatées lors de la mesure d'accélération.
